Europäisches Patentamt

European Patent Office

Office européen des brevets

(11) Veröffentlichungsnummer: **0 388 480**

**A1**

## (12) EUROPÄISCHE PATENTANMELDUNG

(21) Anmeldenummer: **89104922.3**

(22) Anmeldetag: **20.03.89**

(51) Int. Cl.5: **A61N 1/05, A61L 27/00**

(43) Veröffentlichungstag der Anmeldung:
**26.09.90 Patentblatt 90/39**

(84) Benannte Vertragsstaaten:
**CH DE FR GB IT LI NL SE**

(71) Anmelder: **Siemens Aktiengesellschaft**
**Wittelsbacherplatz 2**
**D-8000 München 2(DE)**

(72) Erfinder: **Weidlich, Erhard, Dr. Dipl.-Chem.**
**Am Tennenbach 41**
**D-8521 Spardorf(DE)**
Erfinder: **Lager, Waltraud**
**Hofmannstrasse 11B**
**D-8520 Erlangen(DE)**

(54) **Implantierbare Reizelektrode.**

(57) Bei einer implantierbaren porösen Reizelektrode, insbesondere für Herzschrittmacher, die ein Steroid in Depotform enthält, ist der postoperative Reizschwellenanstieg dann erniedrigt und das Bindegewebswachstum verringert, ohne daß eine Verringerung der Kapazität erfolgt, wenn die Elektrodenoberfläche einen dünnen Überzug aus einem hydrophilen Polymeren aufweist und in das Polymere ein entzündungshemmendes Steroid eingebettet ist.

EP 0 388 480 A1

# Implantierbare Reizelektrode

Die Erfindung betrifft eine implantierbare poröse Reizelektrode, insbesondere für Herzschrittmacher, die ein Steroid in Depotform enthält.

Bei implantierten Reizelektroden ist der Einfluß bzw. die Höhe der Reizschwelle von Bedeutung für die Effektivität der Reizbehandlung. Die Reizschwell wird dabei durch die minimale Spannung, bei der ein Reiz ausgelöst wird, bestimmt bzw. durch den minimalen Strom, bei dem eine Reizwirkung ausgeübt wird, die zur Erregung bzw. Kontraktion der Muskulatur führt. Es hat sich nun gezeigt, daß konventionelle Elektrodensysteme für die Herzschrittmachertherapie nach der Implantation - während der folgenden zwei Wochen - einen Reizschwellenanstieg zeigen. Dies ist darauf zurückzuführen, daß beim Einbringen der Reizelektrode in das Trabekelwerk des Herzens eine Schädigung der Herzmuskulatur erfolgt, was zu einem Bindegewebswachstum führt.

Es ist bereits eine mit Platin beschichtete Titanelektrode bekannt, bei der hinter der porösen Elektrodenoberfläche ein Pfropfen ("plug") aus Silicongummi liegt, der eine geringe Menge (<1 mg) an Dexamethason-natriumphosphat, einem Steroid, enthält (siehe: Society for Biomaterials "Transactions 13th Annual Meeting", June 3-7, 1987, New York, Seite 52, sowie "PACE", Vol. 11 (1988), Seiten 214 bis 219). Nach der Implantation, d.h. nachdem die Elektrode der Körperflüssigkeit ausgesetzt worden ist, wird das Steroid nach und nach freigesetzt, d.h. eluiert, wodurch die Wirksamkeit der Elektrode erhöht werden soll; nach zwei Jahren sollen sich dabei noch 80 % des Steroids in der Elektrode befinden (und - extrapoliert - 18 % nach 100 Jahren).

Bei der bekannten Elektrode steht das Steroid aber nicht nur zeitlich, sondern - bedingt durch den Elektrodenaufbau - auch örtlich nur begrenzt zur Verfügung. Außerdem hat sich gezeigt, daß organische Siliconverbindungen bzw. Siliconbindemittel durch Adsorption an der Oberfläche aktivierter poröser Elektroden einen negativen Einfluß ausüben können, indem sie die Elektrodenoberfläche hydrophobieren und dadurch die Doppelschichtkapazität vermindern.

Aufgabe der Erfindung ist es, eine ein Steroid enthaltende implantierbare Reizelektrode in der Weise auszugestalten, daß zwar einerseits der postoperative Reizschwellenanstieg erniedrigt und das Bindegewebswachstum verringert wird, andererseits aber keine Verringerung der Kapazität erfolgt. Außerdem soll das Steroid während einer kurzen zeitlichen Phase - unmittelbar nach Implantation der Reizelektrode - wirksam werden und dabei soll das umliegende Gewebe gleichmäßig versorgt werden.

Dies wird erfindungsgemäß dadurch erreicht, daß die Elektrodenoberfläche einen dünnen Überzug aus einem hydrophilen Polymeren aufweist und daß in das Polymere ein entzündungshemmendes Steroid eingebettet ist.

Bei der erfindungsgemäßen Reizelektrode ist die poröse Elektrodenoberfläche mit einer dünnen Schicht aus einem polymeren Kunststoff versehen, in die ein Steroid eingelagert ist, das aus dieser Schicht zum angrenzenden Gewebe diffundiert. Dadurch wird der Entzündungsprozeß unterdrückt und der Einheilungsprozeß der Elektrode im Trabekelwerk des Herzmuskels unterstützt, d.h. positiv beeinflußt. Durch eine dosierte Abgabe des Steroids im Bereich um den Elektrodenkopf, wobei auch eine gleichmäßige Versorgung des umgebenden Gewebes auf kurzem Weg erfolgt, d.h. das Steroid wird nicht nur örtlich begrenzt angeboten, wird das Bindegewebswachstum verringert und dadurch der postoperative Reizschwellenanstieg vermindert. Dabei steht das Steroid sofort dann zur Verfügung, wenn es benötigt wird, d.h. unmittelbar nach der Implantation; bereits innerhalb von zwei Tagen ist das Steroid nahezu vollständig eluiert. Durch die erfindungsgemäßen Maßnahmen wird gleichzeitig die poröse Oberfläche der Elektrode vor Verunreinigungen geschützt. Ein Kapazitätsverlust der porösen Elektrodenoberfläche tritt dabei jedoch nicht auf.

Die Schicht aus dem hydrophilen Polymeren und dem darin eingelagerten Steroid ist im allgemeinen etwa 2 bis 50 $\mu$m dick; vorteilhaft beträgt die Schichtdicke 5 bis 10 $\mu$m. Das Polymere ist dabei vorzugsweise ein sulfoniertes Polytetrafluorethylen. Ein derartiges Material, das ionenleitende Eigenschaften aufweist, ist unter dem Namen Nafion im Handel erhältlich. Nafion, das hydrophile und gelartige Eigenschaften besitzt, ist chemisch und thermisch stabil und hat sich bei histologischen Untersuchungen als sehr gut gewebe- und körperverträglich erwiesen.

Als hydrophile Polymere können auch Polyvinylalkohol, Polyacrylsäure, Polyamide, Hydrogele und Cellulose oder Cellulosederivate, wie Acetylcellulose, dienen. Vorteilhaft kann dabei die Einkapselung des Steroids mittels eines Gemisches aus Polyvinylalkohol und Polyacrylsäure erfolgen.

Aus der DE-OS 28 42 318 ist ein implantierbare Kohlenstoffelektrode bekannt, die insbesondere als Reizelektrode dient, beispielsweise für Herzschrittmacher. Bei dieser Elektrode weist die Elektrodenoberfläche einen glatten Überzug aus hydrophilem ionenleitendem Kunststoff auf, wobei wenigstens die Oberfläche des Kunststoffüberzuges aus körper- bzw. blutverträglichem Material besteht. Durch den Kunststoffüberzug sollen die durch einen postoperativen Reizschwellenanstieg in Herzmuskel auftretenden Energieverluste möglichst weitgehend vermieden werden und es soll eine Thrombenbildung an der Elektrodenoberfläche, die zu erhöhten Anfangs- und Dauerreizschwellen führt, verhindert werden.

Als entzündungshemmendes Steroid kann bei der erfindungsgemäßen Reizelektrode vorteilhaft Cortison Verwendung finden. Cortison (gebräuchliche Bezeichnung für $17\alpha.21$-Dihydroxy-4-pregnen-3.11.20-trion) ist eines der wichtigsten Corticosteroide, d.h. Nebennierenrindenhormone. Cortison zählt zu den Glucocorticoiden und weist eine therapeutisch verwertbare Antiphlogistika-Wirkung auf. Ein Potenzierung der glucocorticoiden Wirkung zeigen Derivate des Cortisons, die synthetisch hergestellt werden, wie Prednison und Prednisolon.

Vorzugsweise dient als entzündungshemmendes Steroid Dexamethason oder eines seiner Derivate. Dexamethason (Kurzbezeichnung für $9\alpha$-Fluor-$11\beta.17\alpha.21$-trihydroxy-$16\alpha$-methylpregna-1.4-dien-3.20-dion) weist eine glucocorticoide Wirkung auf und dient unter anderem als Antiphlogistikum. Als Derivate kommen beispielsweise Dexamethason-21-orthophosphat und dessen Dinatriumsalz in Betracht.

Das Polymere bzw. die Polymerschicht kann - neben dem Steroid -vorteilhaft ein Prolamin enthalten. Prolamine, die hydrophil sind, sind Pflanzenproteine, die beispielsweise in Getreide und Mais vorkommen. Als Prolamin dient vorzugsweise Zein, das Hauptprotein aus dem Maiskleber. Zein, das unter anderem die Aminosäuren Glutaminsäure, Leucin, Prolin und Alanin enthält, findet in der Medizin bei Heilungsprozessen Verwendung. Mit Stoffen wie Zein, die im Körper, d.h. im Blut oder Gewebe, wieder abgebaut werden, soll eine schnell einsetzende Wirkung des Steroids erreicht werden.

Bei der erfindungsgemäßen Reizelektrode besteht vorteilhaft wenigstens die Oberfläche des Elektrodenkopfes aus oberflächlich aktiviertem Glas- oder Pyrokohlenstoff. Implantierbare Elektroden aus Glaskohlenstoff und aus Pyrokohlenstoff bzw. Pyrographit sind bekannt (DE-OS 26 13 072 und DE-OS 26 13 052). Derartige Elektroden weisen eine hohe mechanische Stabilität auf und verbrauchen auch im Langzeitbetrieb wenig Energie. Die dabei an glatten Elektroden auftretenden relativ hohen Polarisationsverluste lassen sich vermeiden, wenn die Elektroden bzw. der Elektrodenkopf, d.h. allgemein der aktive Bereich, oberflächlich aktiviert sind und dadurch eine Oberfläche mit mikroporöser Struktur aufweisen. Hierdurch wird nämlich eine hohe Doppelschichtkapazität erreicht.

Vorteilhaft kann die erfindungsgemäße Reizelektrode auch aus einem elektrisch leitenden Trägermaterial bestehen und im aktiven Bereich eine poröse Schicht aus einem Carbid, Nitrid oder Carbonitrid wenigstens eines der Metalle Titan, Vanadium, Zirkonium, Niob, Molybdän, Hafnium, Tantal oder Wolfram aufweisen; bevorzugt wird dabei Titannitrid. Eine derartige Elektrode, die für medizinische Anwendungen dient, ist aus der DE-OS 33 00 668 bekannt. Auch diese Elektrode besitzt eine hohe Doppelschichtkapazität.

Die Oberfläche der erfindungsgemäßen Reizelektrode kann vorteilhaft strukturiert sein. Die Strukturierung kann sich dabei in Form von Rillen, Bohrungen, Kanälen oder andersartigen Vertiefungen ausdrücken. Durch die Veränderung der Elektrodenstruktur wird die Depotwirkung des Polymer/Steroid-Überzuges erhöht.

Anhand von Beispielen soll die Erfindung noch näher erläutert werden.

Beispiel 1: Titannitrid-Reizelektroden

Elektroden aus Titan mit einer aufgesputterten porösen TiN-Schicht (siehe dazu: DE-OS 32 10 420) werden zunächst in Isopropanol und in Aceton gereinigt (Dauer: je 30 min) und dann kurz mit siedendem Ethanol und nachfolgend mit siedendem Isopropanol behandelt. Die derart gereinigten porösen TiN-Reizelektroden werden anschließend kurz entweder in eine Nafion-Dexamethason-Suspension oder in eine mit Dexamethason gesättigte Nafionlösung getaucht. Diese Lösung bzw. Suspension wird folgendermaßen hergestellt.

In einer käuflichen etwa 5 %igen Nafionlösung (in einem Gemisch von niedrigen aliphatischen Alkoholen und Wasser) können bis zu 3,5 % Dexamethason gelöst werden. Wird in die Nafionlösung ein höherer Anteil an Dexamethason eingebracht, so erhält man eine weiße Suspension. Eine derartige Suspension wird beispielsweise aus 10 ml der schwach sauren Nafionlösung und 1 g Dexamethason hergestellt. Um den Säuregrad (bei der Temperaturbehandlung der Elektroden nach der Beschichtung) nicht zu erhöhen, wird die Nafionlösung vor der Beschichtung - und vor der Zugabe des Steroids - mit einer konzentrierten Ammoniaklösung auf einen pH-Wert von 7 bis 8 eingestellt.

Die Reizelektroden werden in eine derartige Lösung bzw. Suspension getaucht, wobei der Tauchvorgang dreimal wiederholt wird. Zwischen den Tauchvorgängen werden die Elektroden jeweils auf etwa 70° C erwärmt. Nach der Beschichtung werden die Reizelektroden zur Trocknung einer Temperaturbehandlung unterworfen: je 1 h bei 70° C, 90° C und 110° C. Nach der Trocknung werden die Elektroden 1 h in physiologischer Kochsalzlösung (0,15 M NaCl, pH = 7) gekocht, um den Quellungsvorgang des Nafions zu beschleunigen. Anschließend werden die Elektroden noch sterilisiert, und zwar 20 min bei 130 °C in physiologischer Kochsalzlösung in verschlossenen Ampullen.

Impedanzmessungen (bei 1 Hz, 100 Hz und 300 Hz) an derart beschichteten Reizelektroden zeigen, daß die Kapazität durch die Beschichtung nicht verringert wird. Bei der Frequenz von 1 Hz ergeben sich sogar geringfügig verbesserte Kapazitätswerte.

Bei der Implantation derartiger Reizelektroden im Herz von Hunden ergeben sich - nach dem Einheilungsprozeß - innerhalb der ersten 50 Tage Reizschwellen von etwa 1 V bzw. 1 bis 1,2 V, jeweils bei 0,5 ms Impulsdauer. Der erste Wert gilt dabei für Elektroden mit einem relativ geringen Steroidgehalt (Verwendung einer Beschichtungslösung), der zweite Wert für Elektroden mit einem Gehalt von etwa 1 mg Dexamethason (Verwendung einer Beschichtungssuspension), jeweils bei einer Elektrodenfläche von 0,12 cm².

Beispiel 2: Glaskohlenstoff-Reizelektroden

a) Nafion/Dexamethason-Beschichtung
Aktivierte Glaskohlenstoff-Elektroden, d.h. Elektroden mit mikroporöser Oberfläche, die zum Teil eine - durch Laserstrahlbehandlung erzeugt - rillenförmige Oberflächenstruktur aufweisen, werden dreimal in die in Beispiel 1 beschriebene Nafion/Dexamethason-Lösung getaucht; zwischen den Tauchvorgängen werden die Elektroden unter Vakuum getrocknet. Nach der Beschichtung werden die Elektroden je 1 h bei 70 °C, 90 °C und 110 °C getempert.

b) Nafion/Dexamethason/Zein-Beschichtung
In einem Lösungsmittelgemisch aus 80 ml Ethanol und 20 ml Wasser werden 2,5 g Dexamethason und 5 g Zein gelöst. 2 ml der dabei erhaltenen Lösung werden mit 2 ml der 5 %igen Nafionlösung gemischt. Mit dieser Lösung werden aktivierte Glaskohlenstoff-Elektroden - wie unter (a) beschrieben - beschichtet und dann getempert.

c) Polyvinylalkohol/Polyacrylsäure/Dexamethason-Beschichtung
Jeweils 5 %ige wäßrige Lösungen von Polyvinylalkohol (PVA) und Polyacrylsäure (PAS) werden im Verhältnis 8:1 gemischt. Zu der dabei erhaltenen Lösung wird - im Verhältnis 1:1 -eine 0,5 %ige Lösung von Dexamethason in Propanol gegeben. Mit dieser Lösung werden aktivierte Glaskohlenstoff-Elektroden - wie unter (a) beschrieben - beschichten und dann getempert.

d) Acetylcellulose/Dexamethason-Beschichtung
Acetylcellulose wird bei Raumtemperatur in Aceton zu einer 5 %igen Lösung gelöst. In 2 ml dieser Lösung werden 5% Dexamethason eingebracht. Mit der dabei erhaltenen Lösung werden aktivierte Glaskohlenstoff-Elektroden - wie unter (a) beschrieben - beschichtet und dann 2 h bei 50 °C getempert.

Vor der Implantation werden die in der vorstehend beschriebenen Weise hergestellten Reizelektroden noch mit Dampf oder mit Ethylenoxid sterilisiert. Versuche haben gezeigt, daß eine Gassterilisation mit Ethylenoxid keine negativen Auswirkungen hat, und zwar weder auf den Kunststoffüberzug noch auf die Elektrodenoberfläche.

Zur Ermittlung der Kapazität wurden die sterilisierten Reizelektroden in einen Elektrolyten (0,15 M NaCl, pH = 7) eingetaucht, und dann wurde die Elektrodenimpedanz als Funktion der Zeit bestimmt (bei 40 °C und 300 Hz). Dabei zeigte sich, daß die Elektroden bereits unmittelbar nach dem Eintauchen 50 % des Maximalwertes der Kapazität aufweisen und somit betriebsbereit sind. Anschließend steigt dann die Kapazität weiter an, weil der Elektrolyt in den Polymerüberzug eindiffundiert, und nach ca. 2 h stellt sich ein stabiler Wert ein, der im wesentlichen dem Maximalwert entspricht, der an unbeschichteten Elektroden ermittelt wird.

Bei der Implantation derartiger Reizelektroden in Schafen stellt sich von Beginn an eine Reizschwelle von ca. 0,6 V ein (bei 0,5 ms Impulsdauer). Dieser Wert ist auch nach 100 Tagen noch unverändert (Steroidgehalt < 1 mg, bei einer Elektrodenfläche von 0,12 cm²).

## Ansprüche

1. Implantierbare poröse Reizelektrode, insbesondere für Herzschrittmacher, enthaltend ein Steroid in Depotform, **dadurch gekennzeichnet,** daß die Elektrodenoberfläche einen dünnen Überzug aus einem hydrophilen Polymeren aufweist und daß in das Polymere ein entzündungshemmendes Steroid eingebettet ist.

2. Implantierbare poröse Reizelektrode nach Anspruch 1, **dadurch gekennzeichnet,** daß das Polymere ein sulfoniertes Polytetrafluorethylen ist.

3. Implantierbare poröse Reizelektrode nach Anspruch 1, **dadurch gekennzeichnet,** daß das Polymere ein Gemisch aus Polyvinylalkohol und Polyacrylsäure ist.

4. Implantierbare poröse Reizelektrode nach einem der Ansprüche 1 bis 3, **dadurch gekennzeichnet,** daß das Steroid Cortison ist.

5. Implantierbare poröse Reizelektrode nach einem der Ansprüche 1 bis 3, **dadurch gekennzeichnet,** daß das Steroid Dexamethason oder ein Derivat dieser Verbindung ist.

6. Implantierbare poröse Reizelektrode nach einem oder mehreren der Ansprüche 1 bis 5, **dadurch gekennzeichnet,** daß das Polymere ein Prolamin enthält.

7. Implantierbare poröse Reizelektrode nach Anspruch 6, **dadurch gekennzeichnet,** daß das Prolamin Zein ist.

8. Implantierbare poröse Reizelektrode nach einem oder mehreren der Ansprüche 1 bis 7, **dadurch gekennzeichnet,** daß wenigstens die Oberfläche des Elektrodenkopfes aus oberflächlich aktiviertem Glas- oder Pyrokohlenstoff besteht.

9. Implantierbare poröse Reizelektrode nach einem oder mehreren der Ansprüche 1 bis 7, **dadurch gekennzeichnet,** daß sie aus einem elektrisch leitenden Trägermaterial besteht und im aktiven Bereich eine poröse Schicht aus einem Carbid, Nitrid oder Carbonitrid wenigstens eines der Metalle Titan, Vanadium, Zirkonium, Niob, Molybdän, Hafnium, Tantal oder Wolfram aufweist, insbesondere aus Titannitrid.

10. Implantierbare poröse Reizelektrode nach einem oder mehreren der Ansprüche 1 bis 9, **dadurch gekennzeichnet,** daß die Elektrodenoberfläche strukturiert ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 304 591 (MUELLER) <br> * Spalte 2, Zeilen 26-46; Spalte 2, Zeile 66 - Spalte 3, Zeile 13 * <br> --- | 1,4,5 | A 61 N 1/05 <br> A 61 L 27/00 |
| Y | US-A-4 506 680 (STOKES) <br> * Spalte 1, Zeile 60 - Spalte 2, Zeile 11; Spalte 3, Zeilen 6-26 * <br> --- | 1,4,5,9 ,10 | |
| Y | EP-A-0 057 451 (MEDTRONIC) <br> * Insgesamt * <br> --- | 1,4,5,9 ,10 | |
| A· | EP-A-0 207 624 (FOX) <br> * Seite 3, Zeilen 7-15; Seite 5, Zeile 26 - Spalte 6, Zeile 4 * <br> --- | 2 | |
| A | EP-A-0 028 122 (UNITIKA) <br> * Seite 3, Absatz 3; Seite 5, Zeilen 1-12; Seite 23, Anspruch 26 * <br> --- | 1,3,8,9 | |
| A | WO-A-8 303 967 (HÄRLE) <br> * Seite 5, Zeilen 5-13 * <br> --- | 6,7 | **RECHERCHIERTE SACHGEBIETE (Int. Cl.5)** |
| A | EP-A-0 242 672 (DINGELDEIN) <br> * Spalte 3, Zeilen 14-26 * <br> --- | 6,7 | A 61 N <br> A 61 L |
| A | EP-A-0 047 013 (MEDTRONIC) <br> * Seite 1, Zeilen 28-36; Seite 2, Zeilen 14-35; Seite 6, Zeilen 1-10; Seite 7, Zeilen 14-15; Seite 8, Zeilen 1-3 * <br> --- | 1,9,10 | |
| A,D | DE-A-2 842 318 (RICHTER) <br> * Spalte 4, Zeilen 13-35 * <br> ---         -/- | 1,3,8, 10 | |

Der vorliegende Recherchenbericht wurde für alle Patentansprüche erstellt

| Recherchenort | Abschlußdatum der Recherche | Prüfer |
|---|---|---|
| DEN HAAG | 21-11-1989 | LEMERCIER D.L.L. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
..................................................................................
& : Mitglied der gleichen Patentfamilie, übereinstimmendes
    Dokument

EPO FORM 1503 03.82 (P0403)

Europäisches
Patentamt

**EUROPÄISCHER RECHERCHENBERICHT**

Nummer der Anmeldung

EP 89 10 4922

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | PACE, vol. 11, February 1988, pages 214-219; H. MOND et al.: "The porous titanium steroid eluting electrode: A double blind study assessing the stimulation threshold effects of steroid"<br>----- | 1,5,9, 10 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| DEN HAAG | 21-11-1989 | LEMERCIER D.L.L. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
 
& : Mitglied der gleichen Patentfamilie, übereinstimmendes
    Dokument

EPO FORM 1503 03.82 (P0403)